# EUROPÄISCHE PATENTSCHRIFT

(11) **EP 3 528 722 B1**
(45) Veröffentlichungstag und Bekanntmachung des Hinweises auf die Patenterteilung: **25.05.2022**
(21) Anmeldenummer: 17792017.0
(22) Anmeldetag: 24.10.2017
(51) Int. Cl.: A61B 17/322, A61B 17/00

(54) **VORRICHTUNG ZUM ENTFETTEN EINES VOLLHAUTTRANSPLANTATS**
DEVICE FOR DEGREASING A FULL-THICKNESS SKIN TRANSPLANT
DISPOSITIF DE DÉGRAISSAGE D'UN GREFFON DE PEAU ENTIÈRE

(30) Priorität: 24.10.2016 DE 102016120220
(43) Veröffentlichungstag der Anmeldung: 28.08.2019
(73) Patentinhaber: Loff, Steffan, 70184 Stuttgart (DE); MAJA-Maschinenfabrik Hermann Schill GmbH, 77694 Kehl-Goldscheuer (DE)
(72) Erfinder: LOFF, Steffan, 77694 Kehl-Goldscheuer (DE)
(74) Vertreter: DREISS Patentanwälte PartG mbB
(86) Internationale Anmeldenummer: PCT/EP2017/077198
(87) Internationale Veröffentlichungsnummer: WO 2018/077897

(56) Entgegenhaltungen:
- CN-A- 104 116 549
- DE-A1- 3 238 255
- US-A- 2 590 299
- US-A- 4 098 278
- US-A1- 2001 047 177
- US-A1- 2005 101 972

## Beschreibung

Schwere Verbrennungen und Verbrühungen führen ab einem Grad von "tief IIb" bis "IV" unabhängig von der Güte der Erstversorgung regelmäßig zur Ausbildung von Narben. Insbesondere über Gelenken und Arealen großer Beweglichkeit bilden sich oft dicke Narbenstränge, die die Bewegung so stark beeinträchtigen können, dass das betroffene Gelenk vollkommen gebrauchsunfähig wird. Klassische Stellen für diese schwerwiegende Komplikation von Verbrennungen und Verbrühungen sind die Fingergelenke, die Ellbogengelenke, die Schultergelenke, die Kniegelenke und der Hals. An der Hand kann bei tiefen Verletzungen eine komplette Versteifung eintreten. An Schulter- und Ellbogengelenk führen Narbenkontrakturen zur Gebrauchsunfähigkeit des betroffenen Armes. Bei den häufigen Beugekontrakturen im Kniegelenk können betroffene Patienten das Bein nicht strecken und damit nicht mehr laufen. Am Hals entwickelt sich nach tiefen Verletzungen die gefürchtete sternomentale Kontraktur, bei der das Kinn durch Narbenzug auf das Brustbein gezogen und fixiert wird. Dies führt zu teilweise schweren Deformationen des Gesichts, Ektropium der Augen und sogar Fehlstellung der Zähne.

Chirurgisch-technisch lassen sich solche Deformierungen durch geeignete plastische Korrekturen therapieren. Man unterscheidet sogenannte lokale Lappen, freie Lappen und Vollhauttransplantate. Vollhauttransplantate sind die erste Wahl unter den korrektiven Maßnahmen bei Defekten, die durch lokale Lappenplastiken nicht gedeckt werden können. Sie führen bei vollständigem Einheilen in der Regel zu einer Beseitigung der Deformität.

Vollhauttransplantate bestehen aus autologer also körpereigener Haut mit Epidermalstrukturen, wie Haarfollikeln und Schweißdrüsen (Dermis in ganzer Dicke). Sie weisen eine Dicke von 0,8-1,2 mm auf.

Vollhauttransplantate werden gewonnen, in dem man an Stellen redundanten Fettgewebes ein Hautareal der erforderlichen Größe entnimmt. Typische Orte sind die Bauchfalte, die Gesäßfalten und die Handgelenksbeugefurchen. Bei der Entnahme wird eine spindelförmige Hautinzision und Exzision des Hautareales mit dem darunterliegenden Fettgewebe durchgeführt. Die Entnahme wird so groß dimensioniert, dass der resultierende Defekt nach Mobilisation der Wundränder durch Nähte wieder verschlossen werden kann. Die maximale Größe wird beispielsweise bei der Bauchhautentnahme in der Länge limitiert durch die Distanz zwischen den beiden Beckenkämmen und in der Breite durch die soeben noch verschließbare Distanz der Wundränder. Bei großen Vollhauttransplantaten kann diese Größe 30cm x 15 cm erreichen.

Das gewünschte Vollhauttransplantat wird in der Entnahmeregion üblicherweise mit Hilfe einer Schablone markiert und anschließend einschließlich der subkutanen Fettschicht entnommen. Bevor das Vollhauttransplantat auf die zu behandelnde Stelle, die Empfängerregion, aufgebracht und in die Wundränder eingenäht werden kann, muss es komplett entfettet werden, also die subkutane Fettschicht weitestgehend entfernt werden.

Nach dem Einnähen in der Empfängerregion ist das Vollhauttransplantat vorübergehend vollständig von der Durchblutung getrennt. Das Vollhauttransplantat wird vorübergehend ausschließlich durch Diffusion aus darunter liegendem Gewebe ernährt. Nach ca. 5-7 Tagen entstehen neue Kapillareinsprossungen in das Vollhauttransplantat und eine Blutzirkulation bildet sich aus.

Eine Versorgung des Vollhauttransplantats durch Diffusion aus dem Plasma kann nur erfolgen, wenn das Vollhauttransplantat ausreichend dünn ist. Wird die subkutane Fettschicht vor dem Einnähen des Vollhauttransplantats nicht oder nicht ausreichend entfernt, so ist das Vollhauttransplantat zu dick, kann nicht über Diffusion versorgt werden und stirbt ab.

Aus dem eben genannten Grund ist die Entfettung des Vollhauttransplantats nach der Entnahme von grundlegender Bedeutung für den Erfolg einer Transplantation. Dabei ist es besonders vorteilhaft, wenn an einzelnen, über die Erstreckung des Vollhauttransplantats verteilten Stellen kleine Fettbereiche am Vollhauttransplantat belassen werden. Diese bilden nach der Transplantation Keimzellen für die Bildung einer neuen subkutanen Fettschicht.

Herkömmlicherweise wird die subkutane Fettschicht von einem Chirurgen mittels eines Skalpell oder einer Schere manuell von dem Vollhauttransplantat entfernt. Dieses Vorgehen ist sehr zeitaufwändig, was zu einer langen Dauer eines entsprechenden Transplantationseingriffs führt. Zum einen stellt dies eine gesundheitliche Belastung für den Patienten dar und zum anderen resultieren hieraus hohe Kosten für einen derartigen Eingriff, da die Entfettung des Vollhauttransplantats von einem erfahrenen Chirurgen durchzuführen ist.

Aus der DE 32 38 255 A1 ist eine Dermatom bekannt, das mehrere Rollen sowie eine Klinge umfasst.

Die CN 10 411 65 549 (A) beschreibt eine Vorrichtung mit einer oszillierenden Klinge und mehreren miteinander gekoppelten Rollen.

Die US 2005/0101972 A1 und US 4,098,278 beschreiben je ein Dermatom zur Entnahme von Spalthaut.

Die US 2001/0047177 A1 beschreibt ein Gerät zur Aufbereitung eines Vollhauttransplantats.

Aufgabe der vorliegenden Erfindung ist es, eine Vorrichtung bereitzustellen, mit der subkutanes Fettgewebe schnell und zuverlässig weitestgehend vollständig von dem Vollhauttransplantat entfernt werden kann. Mit anderen Worten eine Vorrichtung mit der das Vollhauttransplantat schnell und zuverlässig entfettet werden kann.

Diese Aufgabe wird erfindungsgemäß durch eine Vorrichtung nach Anspruch 1 sowie einer Verwendung nach Anspruch 11 und einem Verfahren nach Anspruch 12 gelöst. Bei einer Vorrichtung zum Entfetten eines Vollhauttransplantats, mit einer Schneideinrichtung zum Abtrennen einer an dem Vollhauttransplantat befindlichen subkutanen Fettschicht, ist vorgesehen, dass die Vorrichtung eine Zugwalze umfasst, die über einen Spalt beabstandet zu einer Schneide der Schneideinrichtung angeordnet ist und ausgebildet ist, um das Vollhauttransplantat durch Rotation der Zugwalze der Schneideinrichtung zuzuführen. Die Schneideinrichtung eine Klinge, die die Schneide aufweist, umfasst, die an einem Klingenhalter, insbesondere lösbar, befestigt ist, dadurch gekennzeichnet, dass die Schneide der Klinge zur Rotationsachse der Zugwalze mit einem Schneidenabstand beabstandet ist und ein naheliegenster Abschnitt der Klinge mit einem minimalen Abstand zur Rotationsachse der Zugwalze beabstandet ist, wobei der Schneidenabstand größer ist als der minimale Abstand.

Im Betrieb der Vorrichtung wird das Vollhauttransplantat mit der daran befindlichen subkutanen Fettschicht auf die Zugwalze aufgebracht. Die Zugwalze wird dann rotiert. Durch die Rotation der Zugwalze wird das Vollhauttransplantat gegen eine Schneide der Schneideeinrichtung geführt. Dabei ist der Spalt zwischen Zugwalze und Schneideinrichtung derart ausgebildet, dass das Vollhauttransplantat durch die Rotation der Zugwalze zwischen Zugwalze und Schneideeinrichtung in den Spalt geführt wird, während die Schneideinrichtung die subkutane Fettschicht von dem Vollhauttransplantat abtrennt. Die abgetrennte subkutane Fettschicht befindet sich dann auf der dem Spalt gegenüberliegenden Seite der Schneideinrichtung und kann entnommen und entsorgt werden.

Bevorzugt ist dabei, wenn die Schneideinrichtung eine Klinge, die die Schneide aufweist, umfasst, die an einem Klingenhalter, insbesondere lösbar, befestigt ist. Die Klinge kann einfach entnommen und ausgetauscht oder gereinigt werden, wenn die Klinge lösbar befestigt ist.

Vorzugsweise ist die Klinge am Klingenhalter spielfrei, insbesondere lösbar, befestigt, vorzugsweise verspannt. Hierdurch ermöglicht die Vorrichtung ein besonders reproduzierbares Betriebsverhalten.

Bevorzugt ist, wenn der Klingenhalter spielfrei gegenüber der Zugwalze angeordnet ist. Vorzugsweise ist der Klingenhalter lösbar an der übrigen Vorrichtung befestigt. Im vorliegenden Zusammenhang ist mit einer spielfreien Befestigung insbesondere eine starre Befestigung gemeint, derart, dass die aneinander befestigten Teile nicht relativ zueinander beweglich sind.

Vorzugsweise weist der Klingenhalter eine der Zugwalze zugewandte Oberfläche auf, die gekrümmt ausgebildet ist. Vorzugsweise ist die der Zugwalze zugewandte Oberfläche des Klingenhalters mit einem Krümmungsradius ausgebildet, der anders, vorzugsweise größer, ist, als der Krümmungsradius der Zugwalze. Vorzugsweise ist der Klingenhalter derart ausgebildet und gegenüber der Zugwalze angeordnet, dass ein Sekundärspalt, der dem Abstand zwischen der Zugwalze und der der Zugwalze zugewandten Oberfläche des Klingenhalters an einer schneidennahen Seite des Klingenhalters entspricht, geringer ist als ein Abstand A an einer schneidenabgewandten Seite des Klingenhalters. Hierdurch ergibt sich ein vorteilhaftes Transportverhalten der Zugwalze.

Vorzugsweise ist der Abstand zwischen der Zugwalze und der der Zugwalze zugewandten Oberfläche des Klingenhalters derart ausgebildet, dass er in Transportrichtung zunimmt.

Vorzugsweise ist die schneidennahe Seite des Klingenhalters über einen Sekundärspalt zur Zugwalze beabstandet, wobei der Sekundärspalt größer ist als der Spalt über den die Schneide zur Zugwalze beabstandet ist.

Vorzugsweise ist die Klinge zwischen dem Klingenhalter und einem Klemmelement verspannt angeordnet. Vorzugsweise ist die Klinge über die Verspannung zwischen dem Klingenhalter und dem Klemmelement an der Vorrichtung lösbar befestigt. Hierdurch kann in einfacher Weise eine lösbare und dennoch spielfreie Befestigung der Klinge realisiert werden.

Vorzugsweise sind die Klinge und der Klingenhalter sowie ggf. das Klemmelement parallel zu einer Rotationsachse der Zugwalze angeordnet.

Vorzugsweise ist die Klinge flächig ausgebildet und weist eine Klingendicke von 0,4mm bis 1mm, vorzugsweise 0,5 bis 0,8mm, insbesondere 0,6mm und 0,75mm, auf.

Vorzugsweise weist die Klinge einen Überstand über den Klingenhalter auf, derart, dass die Schneide zwischen 2mm und 6mm, vorzugsweise zwischen 3mm und 5mm zu dem Klingenhalter beabstandet ist.

Vorzugsweise ist die Klinge flächig ausgebildet und derart zur Zugwalze angeordnet, dass beim Blick entlang der Rotationsachse der Zugwalze eine orthogonal zur Rotationsachse und durch die Rotationsachse verlaufende Linie die Klinge in einem Winkel von 90° schneidet, wobei die Schneide vorzugsweise mindestens 0,5mm, vorzugsweise 1mm, insbesondere 1,5mm zum Schnittpunkt der Linie mit der Klinge beabstandet ist.

Vorzugsweise ist die Vorrichtung zerlegbar. Hierdurch kann die Vorrichtung in zerlegter Form in einfacher Weise in einem Autoklaven gereinigt werden.

Vorzugsweise ist die Vorrichtung rein mechanisch angetrieben. Die Vorrichtung dieser Ausführungsform umfasst also bspw. keinen elektrischen Antrieb.

Vorzugsweise ist die Vorrichtung für einen manuellen Betrieb ausgelegt. Bevorzugt ist, wenn die Zugwalze über eine Handkurbel rotierbar ist. Hierdurch kann der Bediener der Vorrichtung den Vorschub des Vollhauttransplantats genau kontrollieren und anpassen. Der Betrieb der Vorrichtung wird damit besonders zuverlässig und eine versehentliche Beschädigung des Vollhauttransplantats kann vermieden werden.

Alternativ kann die Zugwalze über eine Antriebseinheit angetrieben rotierbar ausgeführt sein, wobei eine Rotationsgeschwindigkeit der Zugwalze über ein Bedienelement im Betrieb der Vorrichtung variierbar ist. Diese Ausführungsform stellt eine angetriebene, also nicht bzw. jedenfalls nicht ausschließlich manuell betätigte Ausführungsform dar. Bei dieser Ausführungsform kann insbesondere eine hohe Reproduzierbarkeit der Rotationsgeschwindigkeit der Zugwalze erreicht werden. Die angetriebene Ausführung kann auch mit der Handkurbel kombiniert werden, beispielsweise ist hierbei ein Umschalten oder auch eine Kombination zwischen den beiden Antriebsarten denkbar. Beispielsweise kann ein Motor eine Grundgeschwindigkeit der Zugwalze garantieren, welche manuell durch Betätigung der Handkurbel temporär erhöht werden kann.

Von Vorteil ist außerdem, wenn die Schneide der Schneideinrichtung im Wesentlichen parallel zu der Rotationsachse der Zugwalze angeordnet ist, vorzugsweise wobei der Spalt, über den die Schneide zur Zugwalze beabstandet ist, über die Erstreckung der Zugwalze entlang der Rotationsachse eine gleichbleibende Spaltweite aufweist. Hierdurch wird ein besonders sauberes und genaues Abtrennen der subkutanen Fettschicht gewährleistet.

Bevorzugt ist, wenn der Spalt zwischen der Schneide der Schneideinrichtung und der Zugwalze eine Spaltweite von 0,8-1,2 mm aufweist. Der Spalt ist damit derart ausgebildet, dass das entfettete Vollhauttransplantat eine Dicke von 0,8-1,2 mm aufweist. Hierdurch kann eine manuelle Nachbearbeitung des Vollhauttransplantats entfallen.

Vorzugsweise lässt sich der Spalt stufenweise verstellen.

Vorteilhaft ist, wenn die Zugwalze auf ihrer Wirkfläche rippenförmige oder knollige Erhebungen aufweist. Vorteilhafterweise sind diese Erhebungen über die gesamte Oberfläche der Zugwalze verteilt angeordnet. Vorzugsweise sind die Erhebungen rippenförmig und verlaufen parallel zu einer Rotationsachse der Zugwalze. In einer bevorzugten Ausführungsform weist die Zugwalze einen Durchmesser von wenigstens 4cm, insbesondere 5 cm, vorzugsweise höchstens 8cm, insbesondere höchstens 7cm, auf und auf der Wirkfläche der Zugwalze sind zwischen 50 und 90, insbesondere zwischen 55 und 80, rippenförmige Erhebungen jeweils gleichmäßig verteilt angeordnet. Vorzugsweise beträgt der Abstand zwischen den einzelnen Erhebungen in Umfangsrichtung 1,5mm bis 3,5mm und besonders bevorzugt zwischen 2,0mm und 3,0mm.

Zum einen führen die genannten Erhebungen zu einer verbesserten Kontaktierung des Vollhauttransplantats durch die Zugwalze und zum anderen wird damit erreicht, das die subkutane Fettschicht nicht über die gesamte Erstreckung des Vollhauttransplantats vollständig entfernt wird, sondern es bleiben kleine Bereiche an subkutanem Fett auf dem Vollhauttransplantat erhalten. Diese kleinen Bereiche an subkutanem Fett können im transplantierten Zustand als Keimzellen für die Bildung einer neuen subkutanen Fettschicht dienen.

Die Zugwalze kann Erhebungen aufweisen, die über Vertiefungen zueinander beabstandet sind. Die Vertiefungen können mit einem kreissegmentförmigen Querschnitt ausgebildet sein. Die Vertiefungen können einen Krümmungsradius aufweisen, der größer ist, als ein Krümmungsradius der Erhebungen. Die Erhebungen können durch die Vertiefungen in umfänglicher Richtung weiter zueinander beabstandet sein als sie in umfänglicher Richtung erstreckt sind.

Vorteilhaft ist auch, wenn auf einer der Zugwalze gegenüberliegenden Seite der Schneide eine Anpresswalze angeordnet ist, die ausgebildet ist, um das Vollhauttransplantat mit daran befindlicher subkutaner Fettschicht gegen die Zugwalze zu pressen. Vorzugsweise ist die Anpresswalze nachgiebig ausgebildet. Hierdurch wird eine besonders genaue Durchführung des Vollhauttransplantats zwischen Spalt und Zugwalze gewährleistet. Das Vollhauttransplantat samt subkutaner Fettschicht wird quasi zwischen der Zugwalze und der Anpresswalze genau positioniert und dann gegen die Schneideeinrichtung geführt. Durch die nachgiebige Ausführung der Anpresswalze können Variationen der Dicke des Vollhauttransplantats samt subkutanem Fettgewebe ausgeglichen werden.

Bevorzugt ist auch, wenn die Spaltweite zwischen der Schneide und der Zugwalze einstellbar ist. Wenn also eine Position der Schneide der Schneideinrichtung gegenüber der Zugwalze einstellbar ist. Hierdurch kann die Dicke des Vollhauttransplantats nach dem Abtrennen der subkutanen Fettschicht je nach Bedarf eingestellt werden. Mit anderen Worten, die Vorrichtung lässt sich derart einstellen, dass ein Teil der subkutanen Fettschicht an dem Vollhauttransplantat erhalten bleibt.

Von Vorteil ist auch, wenn die Vorrichtung zerlegbar ist und jedenfalls die Schneideinrichtung und die Zugwalze, sterilisierbar, insbesondere autoklavierbar, sind. Zum einen müssen die einzelnen Teile der Vorrichtung hierzu auf einfache Weise voneinander lösbar sein. Ein Beispiel für einfach lösbare Verbindungen sind Steckverbindungen. Damit die einzelnen Teile sterilisierbar bzw. autoklavierbar sind, müssen diese möglichst glatt und ohne unzugängliche Stellen oder Ähnliches ausgebildet sein. Beispielsweise in Hinterschneidungen oder ähnlich unzugänglichen Stellen können sich Verschmutzungen anlagern, die über einen normalen Sterilisationsprozess nicht entfernt werden können. Durch die Sterilisierbarkeit wird medizinischen Anforderungen entsprochen.

In einer bevorzugten Ausführungsform weist die Zugwalze jedenfalls bereichsweise eine verrundete Wirkfläche auf, vorzugsweise ist die Zugwalze auf ihrer gesamten Wirkfläche verrundet ausgebildet. Mit Wirkfläche ist dabei die Fläche der Zugwalze gemeint, die mit dem Vollhauttransplantat in Kontakt kommen kann. Verrundet bedeutet dabei, dass die Wirkfläche in dem verrundeten Bereich bzw. vorzugsweise über die gesamte Wirkfläche keine scharfen Kanten aufweist. Vorzugsweise sind sämtliche Kanten auf der Wirkfläche, bzw. auf dem verrundeten Bereich der Wirkfläche, mit einem Krümmungsradius von wenigstens 0,25mm, insbesondere 0,5mm, vorzugsweise 1 mm, verrundet.

Vorteilhaft ist auch, wenn die Zugwalze auf ihrer Wirkfläche wenigstens eine Ansaugöffnung, vorzugsweise eine Vielzahl an Ansaugöffnungen, die über die Wirkfläche verteilt sind, aufweist, die mit einer Unterdruckquelle verbindbar ist bzw. sind. Hierdurch kann das Vollhauttransplantat, wenn es auf die Zugwalze aufgelegt wird, durch die Ansaugöffnung mit Unterdruck an die Zugwalze angesaugt wird. Hierdurch wird stets ein sicherer und rutschsicherer Kontakt zwischen der Zugwalze und dem Vollhauttransplantat sichergestellt.

Vorteilhaft ist, wenn die Klinge schraubenlos an der Vorrichtung, insbesondere an dem Klingenhalter, befestigt ist.

Vorteilhaft ist, wenn die Vorrichtung derart zerlegbar ist, dass ihre Einzelteile spaltfrei ausgebildet sind. Hierdurch sind die Einzelteile einfach zu reinigen.

Vorzugsweise sind einige, vorzugsweise alle Einzelteile hinterschneidungsfrei ausgebildet.

Vorteilhaft ist, wenn die Zugwalze Edelstahl umfasst, vorzugsweise aus Edelstahl besteht.

Teil der vorliegenden Erfindung ist ebenso die Verwendung einer Vorrichtung nach einer oder mehreren der beschriebenen Ausführungsformen zur Entfettung eines Vollhauttransplantats.

Teil der vorliegenden Erfindung ist ebenso ein Verfahren zur Entfettung eines Vollhauttransplantats, wobei das Verfahren die Schritte umfasst:
das Vollhauttransplantat mit der daran befindlichen subkutanen Fettschicht derart auf eine Zugwalze aufbringen, dass das Vollhauttransplantat der Zugwalze zugewandt ist und die subkutane Fettschicht der Zugwalze abgewandt ist;
das Vollhauttransplantat mit der daran befindlichen subkutanen Fettschicht durch Rotation der Zugwalze gegen eine Schneide einer Schneideeinrichtung führen, wobei ein Spalt zwischen Zugwalze und Schneideinrichtung derart ausgebildet ist, dass das Vollhauttransplantat durch die Rotation der Zugwalze zwischen Zugwalze und Schneideeinrichtung in den Spalt geführt wird, während die Schneideinrichtung die subkutane Fettschicht von dem Vollhauttransplantat abtrennt.

Vorzugsweise erfolgt die Rotation der Zugwalze bei dem Verfahren manuell, insbesondere über eine Handkurbel.

Das Verfahren wird vorzugsweise unter Verwendung einer der oben beschriebenen Vorrichtungen durchgeführt. Bei dem Verfahren ist es vorteilhaft, wenn die einzelnen oben beschriebenen Aspekte der Vorrichtung bei dem Verfahren vorgesehen sind.

Insbesondere ist es vorteilhaft, wenn bei dem Verfahren die Schneide der Schneideinrichtung im Wesentlichen parallel zu einer Rotationsachse der Zugwalze angeordnet ist.

Vorteilhaft ist bei dem Verfahren insbesondere, wenn die Zugwalze auf ihrer Wirkfläche rippenförmige oder knollige Erhebungen aufweist. Vorteilhafterweise sind diese Erhebungen über die gesamte Oberfläche der Zugwalze verteilt angeordnet. Vorzugsweise sind die Erhebungen rippenförmig und verlaufen parallel zu der Rotationsachse der Zugwalze.

Die Zugwalze kann insbesondere eine geometrisch strukturierte Oberfläche bzw. Wirkfläche aufweisen. Weitere Merkmale, Anwendungsmöglichkeiten und Vorteile der Erfindung ergeben sich aus der nachfolgenden Beschreibung von Ausführungsbeispielen die anhand der Zeichnung erläutert werden, wobei die Merkmale sowohl in Alleinstellung als auch in unterschiedlicher Kombination wichtig sein können, ohne dass hierauf nochmals explizit hingewiesen wird. Es zeigen:
- Figur 1: eine perspektivische Darstellung einer Vorrichtung;
- Figur 2: eine Schnittdarstellung der Vorrichtung;
- Figur 3: die Vorrichtung der Figuren 1 und 2 im Betrieb in der Darstellung von Figur 2;
- Figur 4: eine alternative Vorrichtung in der Darstellung von Figur 2 und 3;
- Figur 5: eine schematische Einzeldarstellung einer Zugwalze einer alternativen Vorrichtung;
- Figur 6: eine erfindungsgemäße Ausführungsform in verschiedenen Darstellungen;
- Figur 7: eine Schnittdarstellung durch die Zugwalze der erfindungsgemäßen Ausführungsform; und
- Figur 8: einen Teilbereich aus Figur 7 in vergrößerter Darstellung; und
- Figur 9: eine Schnittdarstellung durch eine Zugwalze der erfindungsgemäßen Ausführungsform.

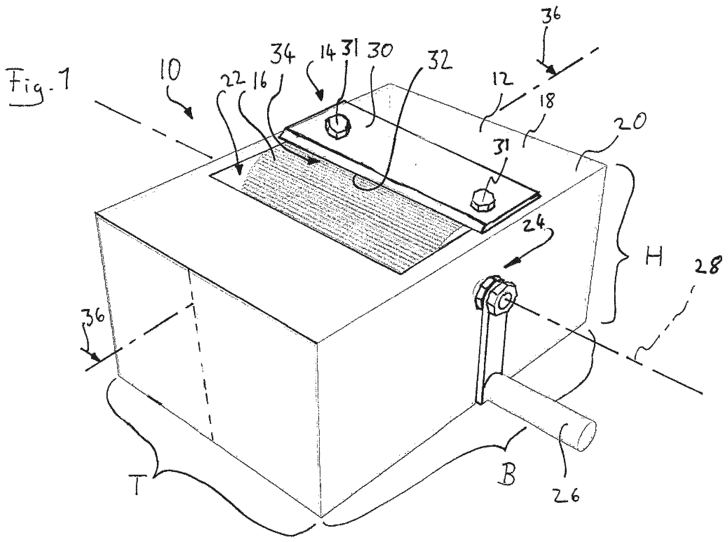

Figur 1 zeigt eine Vorrichtung 10 zum Entfetten eines Vollhauttransplantats. Die Vorrichtung 10 umfasst einen Grundkörper 12, eine Schneideinrichtung 14 und eine Zugwalze 16.

Der Grundkörper 12 besteht vorliegend im Wesentlichen aus einem rechteckigen Gehäuse 18 aus Edelstahl. In der vorliegenden beispielhaften Ausführungsform der Erfindung weist das Edelstahlgehäuse eine Breite B von in diesem Beispiel 30cm, eine Tiefe T von in diesem Beispiel 20cm und eine Höhe H von in diesem Beispiel ebenfalls 20cm auf. Diese Abmessungen sind jedoch keinesfalls einschränkend zu verstehen, sondern sollen lediglich beispielhaft die vorliegend beschriebene Vorrichtung 10illustrieren.

Das Gehäuse 18 weist an einer Oberseite 20 eine Öffnung 22 auf. Beim Blick in die Öffnung 22 ist die im Inneren des Gehäuses 18 angeordnete Zugwalze 16 sichtbar.

Die Zugwalze 16 ist über eine Aufhängung 24 rotierbar mit dem Grundkörper 12 verbunden. Eine Handkurbel 26 ist an der Außenseite des Grundkörpers 12 angebracht. Die Handkurbel ist mit der Zugwalze 16 derart verbunden, dass die Zugwalze 16 über Betätigung der Handkurbel 26 um eine Rotationsachse 28 rotierbar ist.

Im Bereich der Öffnung 22 ist eine Klinge 30 der Schneideinrichtung 14 über zwei Fixier- und Stellelemente 31 derart angeordnet, dass eine Schneide 32 der Klinge 30 über einen Spalt 34 beabstandet zu der Zugwalze 16 angeordnet ist. Die Schneide 32 der Klinge 30 verläuft parallel zur Rotationsachse 28 der Zugwalze 16.

Die Klinge 30 der Schneideinrichtung ist über die beiden Fixier- und Stellelemente 31 an dem Gehäuse angebracht. Neben der Befestigung der Klinge 30 haben die Fixier- und Stellelemente 31 auch die Funktion eines Stellmechanismus für die Schneide 32. Mittels der Fixier- und Stellelemente 31 lässt sich die Anordnung der Klinge 30 bzw. der Spalt 34 zur Zugwalze 16 einstellen und variieren.

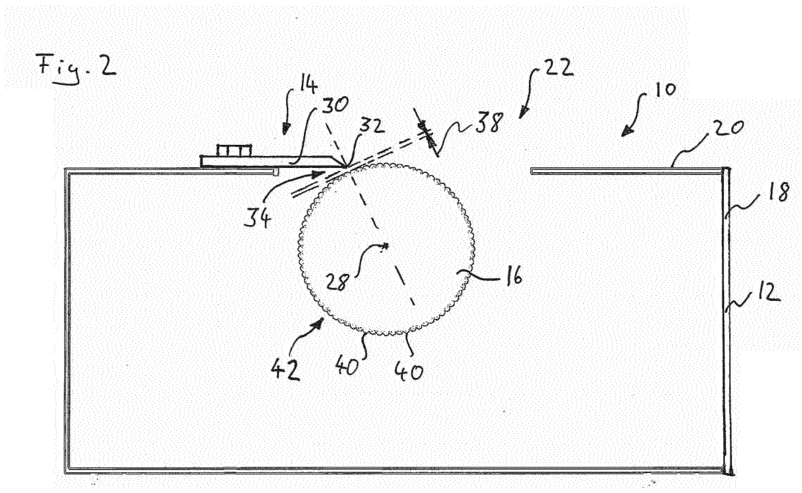

Figur 2 zeigt die Vorrichtung 10 entlang der Linie II-II geschnitten und in Richtung der Pfeile 36 gesehen. In Figur 2 ist insbesondere die über den Spalt 34 beabstandete Anordnung der Zugwalze 16 zur Schneide 32 gut erkennbar. Der Spalt 34 weist eine Spaltweite 38 auf, die vorliegend einen Millimeter beträgt. Die Spaltweite 38 entspricht dem Abstand der Schneide 32 zur Oberfläche der Zugwalze 16.

Wie in Figur 2 erkennbar weist die Zugwalze 16 eine Vielzahl an rippenartigen Erhebungen 40 auf, die auf einer Wirkfläche 42 der Zugwalze 16 angeordnet sind. Wie auch in Figur 1 ersichtlich, erstrecken sich die rippenartigen Erhebungen 40 parallel zur Rotationsachse 28 der Zugwalze 16. Von den Erhebungen 40 sind lediglich zwei in Figur 2 mit einem Bezugszeichen versehen. Die Wirkfläche 42 der Zugwalze 16 entspricht vorliegend der parallel zur Rotationsachse 28 verlaufenden Oberfläche der Zugwalze 16. Mit der Wirkfläche 42 ist derjenige Teil der Oberfläche der Zugwalze 16 gemeint, der dazu vorgesehen ist das Vollhauttransplantat der Schneideinrichtung 14 zuzuführen.

Die rippenförmigen Erhebungen 40 weisen im vorliegenden Beispiel jeweils eine Höhe von ca. 1 mm auf und sind in etwa 1 mm breit. Andere Abmessungen sind denkbar. Die rippenförmigen Erhebungen 40 haben im vorliegenden Beispiel einen im Wesentlichen halbkreisförmigen Querschnitt, wobei der entsprechende Kreis im vorliegenden Beispiel einen Durchmesser von 1 mm aufweist.

Die Spaltweite 38 bemisst sich von der Oberseite der rippenartigen Erhebungen 40 zu der Schneide 32 der Klinge 30.

Die Wirkfläche 42 der Zugwalze 16 ist insgesamt verrundet ausgebildet, damit ist gemeint, dass die Wirkfläche 42 keine scharfen Kanten besitzt. Jeder Bereich, der als Kante interpretiert werden könnte ist verrundet, wobei vorzugsweise die Verrundung einen Radius von wenigstens einen viertel mm, vorzugsweise einen halben mm, aufweist.

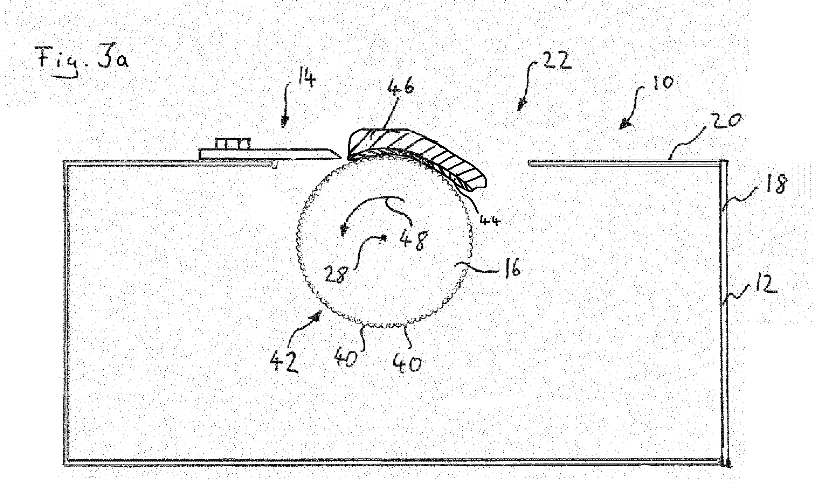

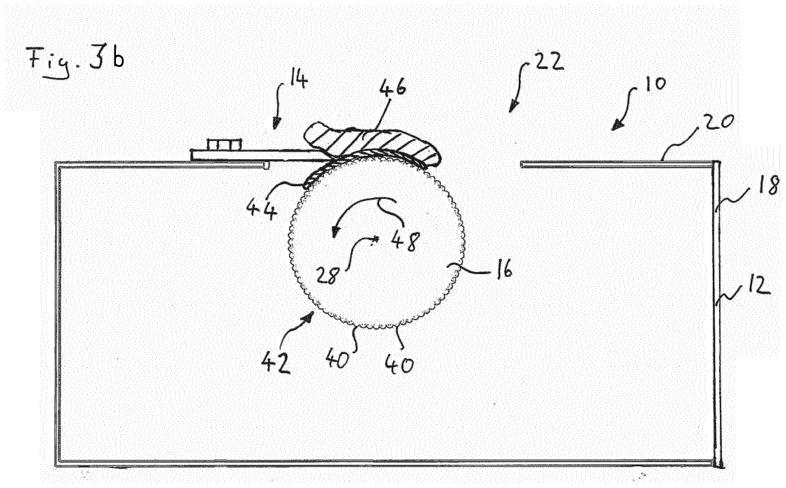

In den Figuren 3a und 3b ist die Vorrichtung 10 aus den Figuren 1 und 2 im Betrieb gezeigt. Außerdem sind Schritte des erfindungsgemäßen Verfahrens gezeigt.

Ein Vollhauttransplantat 44 mit daran befindlichem subkutanem Fettgewebe 46 wird in Figur 3a auf die Zugwalze 16 aufgebracht. Dabei kontaktiert das Vollhauttransplantat 44 die Zugwalze 16 und die subkutane Fettschicht ist der Zugwalze 16 abgewandt.

Die Zugwalze 16 wird über die Handkurbel 26 in Rotation versetzt, was durch einen Pfeil mit dem Bezugszeichen 48 verdeutlicht ist. Das Vollhauttransplantat 44 mit der daran befindlichen subkutanen Fettschicht 46 wird durch die Rotation von der Position aus Figur 3a in die Position aus Figur 3b und weiter bewegt.

Das Vollhauttransplantat 44 mit der daran befindlichen subkutanen Fettschicht 46 wird durch die Rotation der Zugwalze 16 gegen die Schneide 32 der Schneideeinrichtung 14 geführt, wobei ein Spalt 34 zwischen Zugwalze 16 und Schneideinrichtung 14 derart ausgebildet ist, dass das Vollhauttransplantat 44 durch die Rotation der Zugwalze 16 zwischen Zugwalze 16 und Schneideeinrichtung 14 in den Spalt 34 geführt wird, während die Schneideinrichtung 14 die subkutane Fettschicht 46 von dem Vollhauttransplantat 44 abtrennt. Dieses Abtrennen ist in Figur 3b illustriert. Das Vollhauttransplantat 44 mit dem daran befindlichen subkutanen Fettgewebe 46 wird durch die Rotation der Zugwalze 16 gegen die Schneide 32 der Schneideinrichtung 14 geführt. Hierdurch wird das subkutane Fettgewebe 46 von dem Vollhauttransplantat 44 abgetrennt. Ist die komplette Schicht an subkutanem Fettgewebe 46 von dem Vollhauttransplantat 44 abgetrennt, so kann das subkutane Fettgewebe 46 entnommen und entsorgt werden. Auch das Vollhauttransplantat 44 kann entnommen und verwendet werden.

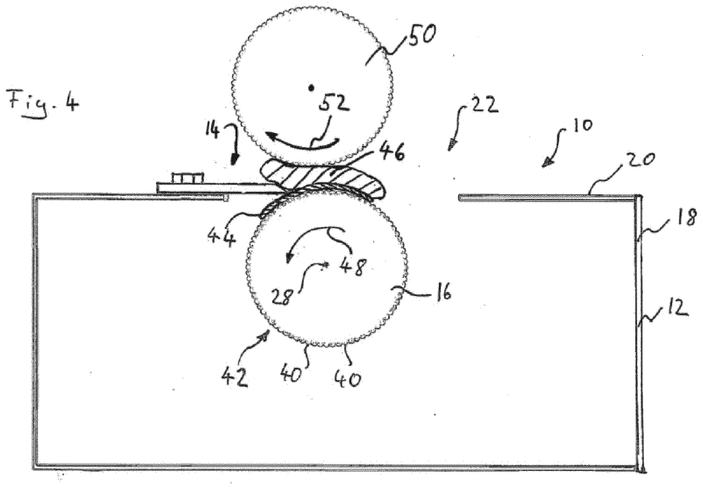

Figur 4 zeigt eine alternative Vorrichtung 10 in einer Darstellung entsprechend den Figuren 2 und 3. Die Vorrichtung 10 nach Figur 4 unterscheidet sich von der Vorrichtung 10 nach den Figuren 1 bis 3 dadurch, dass sie eine Anpresswalze 5 umfasst.

Die Anpresswalze 50 ist auf einer der Zugwalze 16 gegenüberliegenden Seite der Schneide 32 angeordnet. Die Anpresswalze 50 ist ausgebildet und angeordnet, um das Vollhauttransplantat 44 samt daran befindlichem subkutanem Fettgewebe 46 gegen die Zugwalze 16 zu pressen, also anzudrücken. Damit ist lediglich ein leichtes, Richtung vorgebendes Andrücken im Sinne eines Führens gemeint.

Vorteilhafterweise ist der Abstand zwischen Zugwalze 16 und Anpresswalze 50 variierbar. Bevorzugt ist, wenn die Variation des Abstands zwischen Zugwalze 16 und Anpresswalze 50 durch ein Verlagern bzw. ein Verschieben der Anpresswalze 50 vorgenommen werden kann.

Im Betrieb der Vorrichtung 10 gemäß der Ausführungsform von Figur 4 wird das Vollhauttransplantat 44 samt subkutanem Fettgewebe 46 zwischen Anpresswalze 50 und Zugwalze 16 eingeführt. Die Zugwalze 16 wird dann über die Handkurbel 26 in Rotation versetzt. Die Anpresswalze 50 ist passiv gelagert. Damit ist gemeint, dass die Anpresswalze 50 nicht angetrieben ist, sondern lediglich durch den Kontakt mit dem subkutanen Fettgewebe 46 in Rotation versetzt wird, was durch einen Pfeil mit dem Bezugszeichen 52 angedeutet ist. Denkbar ist jedoch auch, dass die Rotation der Anpresswalze 50 an die Rotation der Zugwalze 16, beispielsweise mechanisch, gekoppelt ist. Dabei sind die Oberflächengeschwindigkeiten der Zugwalze 16 und der Anpresswalze 50 vom Betrag her vorzugsweise gleich.

Dadurch, dass das Vollhauttransplantat 44 und das subkutane Fettgewebe 46 zwischen Anpresswalze 50 und Zugwalze 16 quasi eingespannt sind, werden diese durch die Rotation der Zugwalze 16 gegen die Schneide 32 der Schneideinrichtung 14 geführt, wobei der Abstand zwischen Zugwalze 16 und Schneide 32 der Spaltweite 38 entspricht, so dass die Schneide 32 genau zwischen subkutanem Fettgewebe 46 und Vollhauttransplantat 44 ansetzt und diese voneinander trennt.

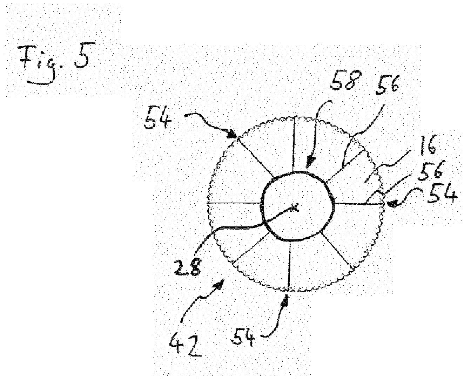

Figur 5 zeigt eine Einzeldarstellung einer Zugwalze 16 einer alternativen Ausführungsform in einer Darstellung entsprechend den Figuren 2 bis 4. Die Zugwalze 16 dieser Ausführungsform weist auf ihrer Wirkfläche 42 eine Vielzahl an über die Wirkfläche 42 verteilten Ansaugöffnungen 54 auf. Die Ansaugöffnungen 54 sind jeweils über Kanäle 56 mit einem sich entlang der Rotationsachse 28 erstreckenden Unterdruckkanal 58 verbunden.

Über den Unterdruckkanal 58 ist die Zugwalze 16 dieser Ausführungsform mit einer Unterdruckquelle, die vorliegend nicht dargestellt ist, verbindbar. Hierdurch können die Ansaugöffnungen 54 mit Unterdruck beaufschlagt werden, so dass diese ein Vollhauttransplantat 44, das auf die Zugwalze 16 dieser Ausführungsform aufgelegt wird, ansaugen und in engem Kontakt mit der Wirkfläche 42 der Zugwalze 16 halten.

Derartige Ansaugöffnungen 54 können ebenso an der Anpresswalze 50 vorgesehen sein, wobei diese dann dazu dienen, die Schicht an subkutanem Fettgewebe 46 gegen die Ansaugwalze 50 zu saugen. Durch das Vorsehen von Ansaugöffnungen 54 sowohl in der Zugwalze 16 als auch in der Anpresswalze 50 kann die Trennwirkung der Schneide 32 erhöht werden.

Denkbar ist auch, dass die einzelnen Kanäle 56 nicht mit einem zentralen Unterdruckkanal 58 verbunden sind, sondern jeweils einzeln mit einer Unterdruckquelle verbindbar sind. Anders ausgedrückt, in dieser Ausführungsform kommunizieren die Kanäle 56 nicht fluidisch miteinander.

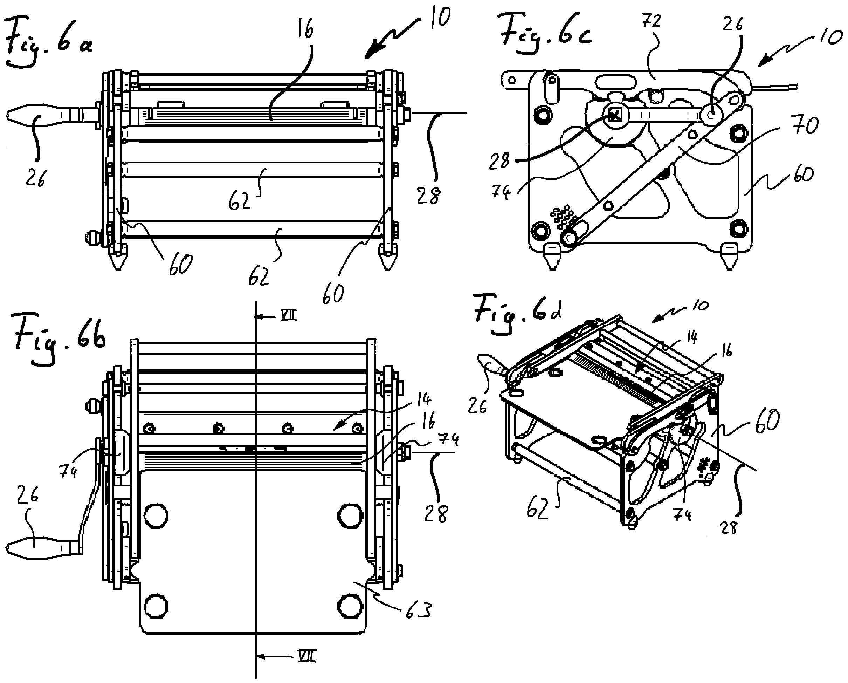

Figur 6 zeigt eine Ausführungsform der erfindungsgemäßen Vorrichtung 10 in verschiedenen Darstellungen (Figur 6a in einer Vorderansicht; Figur 6b in einer Draufsicht; Figur 6c in einer Seitenansicht und Figur 6d in einer perspektivischen Darstellung).

Die Vorrichtung 10 von Figur 6 umfasst einen gestellartigen Grundkörper 12, die Schneideinrichtung 14 und die Zugwalze 16.

Der Grundkörper 12 umfasst im vorliegenden Beispiel zwei plattenartige Seitenelemente 60, die über strebenartige Längselemente 62 miteinander verbunden sind. Die Längselemente erstrecken sich parallel zu der Rotationsachse 28 der Zugwalze 16 und die Seitenelemente 60 hierzu orthogonal.

Die Vorrichtung 10 von Figur 6 umfasst einen optionalen Zuführtisch 63, der gegenüber der Schneideinrichtung 14 angeordnet ist. Der Zuführtisch 63 ist zur Auflage des Vollhauttransplantats 44 mit daran befindlicher subkutaner Fettschicht 46 ausgebildet. Über den Zuführtisch 63 ist das Vollhauttransplantat 44 der Zugwalze 16 zuführbar. Beim Blick entlang der Rotationsachse 28 ist die Zugwalze 16 zwischen dem Zuführtisch 63 und der Schneide 32 angeordnet.

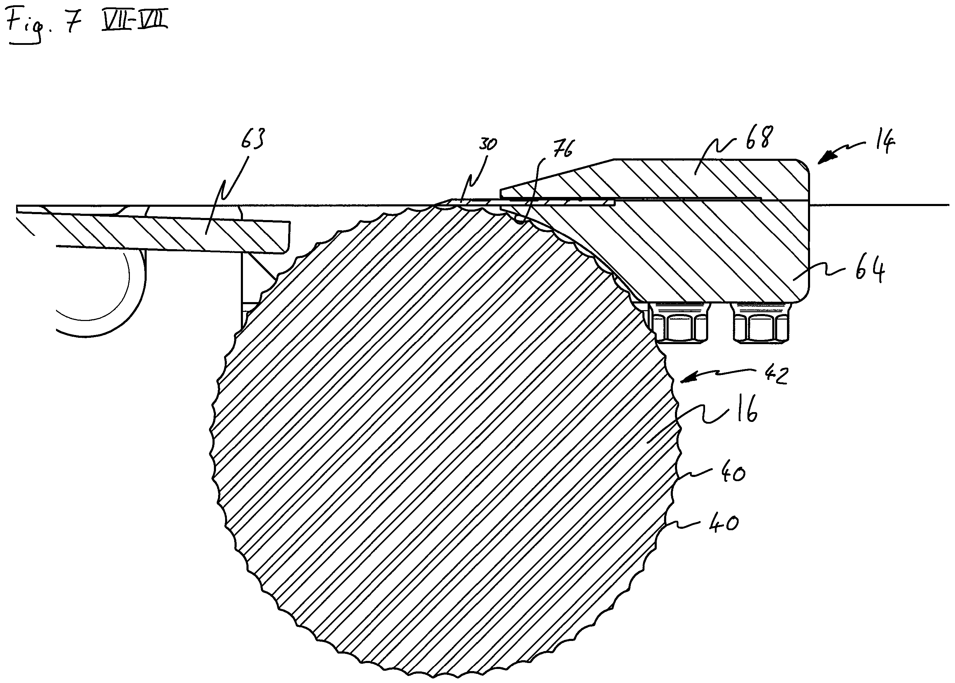

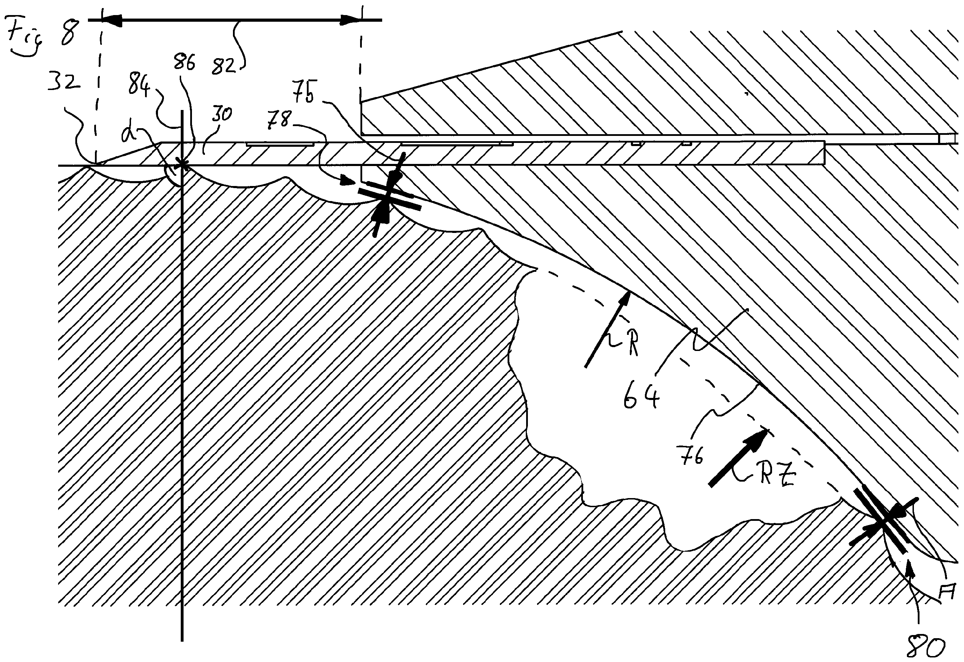

Die Schneideinrichtung 14 der Vorrichtung 10 aus Figur 6 ist in den Figuren 7 und 8 im Detail in einer Schnittdarstellung entlang der Linie VII-VII der Figur 6b gezeigt. In Figur 8 ist die Zugwalze 16 mit einem Ausbruch dargestellt.

Die Schneideinrichtung 14 umfasst einen Klingenhalter 64, die Klinge 30 und ein Klemmelement 68. Die Klinge 30 umfasst die Schneide 32.

Die Klinge 30 ist an dem Klingenhalter 64 lösbar spielfrei befestigt. Vorliegend ist die Befestigung über ein Verklemmen der Klinge 30 zwischen dem Klingenhalter 64 und dem Klemmelement 68 realisiert, die Klinge ist hierdurch am Klingenhalter 64 verspannt. Andere Arten der Befestigung sind denkbar.

Der Klingenhalter 64 ist spielfrei gegenüber der Zugwalze 16 angeordnet. Der Klingenhalter 64 und damit die Schneide 32 kann bzw. können über einen Positionierhebel 70 in ihrer Position, insbesondere in ihrem Abstand ggü. der Zugwalze 16 einstellbar sein. Über ein Verriegelungselement 72 sind der Klingenhalter 64 und die Zugwalze 16 in ihrer jeweiligen Position verriegelt bzw. fixiert. Die Zugwalze 16 ist über Gleitlager 74 aus einem Polymerwerkstoff, vorzugsweise PEEK (Polyetheretherketon)(andere Polymerwerkstoffe sind denkbar), gegenüber den Seitenelementen 60 drehbar gelagert.

Der Klingenhalter 64 weist eine der Zugwalze 16 zugewandte Oberfläche 76 auf, die gekrümmt ausgebildet ist. Die der Zugwalze 16 zugewandte Oberfläche 76 des Klingenhalters 64 ist mit einem Krümmungsradius R ausgebildet, der größer ist als ein Krümmungsradius RZ der Zugwalze 16. Der Klingenhalter 64 ist derart ausgebildet und gegenüber der Zugwalze 16 angeordnet, dass ein Sekundärspalt 75, der dem Abstand zwischen der Zugwalze 16 und der der Zugwalze 16 zugewandten Oberfläche 76 des Klingenhalters 64 an einer schneidennahen Seite 78 des Klingenhalters entspricht, geringer ist als ein Abstand A an einer schneidenabgewandten Seite 80 des Klingenhalters 64. Hierdurch ergibt sich ein vorteilhaftes Transportverhalten der Zugwalze 16. Der Sekundärspalt 75 ist im vorliegend gezeigten Beispiel größer als der Spalt 34. Der Spalt 34 ist in der vorliegenden Darstellung auf 0 eingestellt. Vorzugsweise vergrößert sich beim Vergrößern des Spalts 34 auch der Sekundärspalt 75 bzw. der Abstand zwischen der Zugwalze 16 und der der Zugwalze 16 zugewandten Oberfläche 76 des Klingenhalters 64 über die gesamte Erstreckung der Oberfläche 76.

Vorzugsweise ist der Abstand zwischen der Zugwalze 16 und der der Zugwalze 16 zugewandten Oberfläche 76 des Klingenhalters 64 derart ausgebildet, dass er in Transportrichtung, also Rotationsrichtung der Zugwalze 16 im Betrieb der Vorrichtung 10, zunimmt.

Vorzugsweise weist die Klinge 30 einen Überstand 82 über den Klingenhalter 64 auf, derart, dass die Schneide 32 zwischen 2mm und 6mm, vorzugsweise zwischen 3mm und 5mm zu dem Klingenhalter 64 beabstandet ist.

Die Klinge 30 ist vorliegend flächig ausgebildet und derart zur Zugwalze 16 angeordnet, dass beim Blick entlang der Rotationsachse der Zugwalze 16 (Darstellung von Figur 7 und 8) eine orthogonal zur Rotationsachse 28 und durch die Rotationsachse 28 verlaufende Linie 84 die Klinge 30 in einem Winkel α von 90° schneidet, wobei die Schneide 32 vorzugsweise mindestens 0,5mm, vorzugsweise 1mm, insbesondere 1,5mm zum Schnittpunkt 86 der Linie 84 mit der Klinge 30 beabstandet ist.

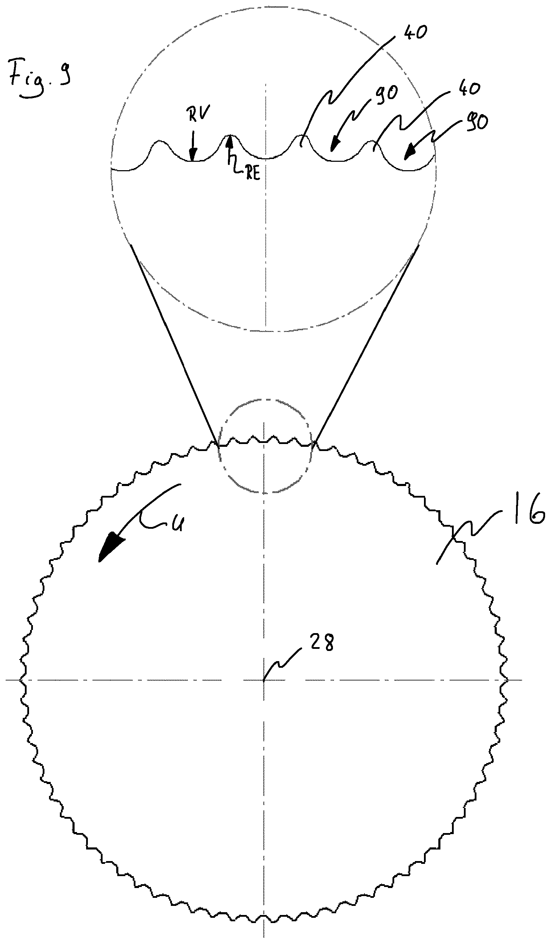

Figur 9 zeigt eine alternative Ausführungsform der Zugwalze 16 in einem Schnitt orthogonal zur Drehachse 28. Die Zugwalze weist die Erhebungen 40 auf, die über Vertiefungen 90 zueinander beabstandet sind. Die Vertiefungen 90 sind im vorliegenden Beispiel mit einem kreissegmentförmigen Querschnitt ausgebildet. Die Vertiefungen 90 können, wie in Figur 9 gezeigt, einen Krümmungsradius RV aufweisen, der größer ist, als ein Krümmungsradius RE der Erhebungen. Im vorliegenden Beispiel sind die Erhebungen 40 durch die Vertiefungen 90 in umfänglicher Richtung U weiter zueinander beabstandet als sie in umfänglicher Richtung U erstreckt sind.

## Patentansprüche

1. Vorrichtung (10) zum Entfetten eines Vollhauttransplantats (44), mit einer Schneideinrichtung (14), die zum Abtrennen einer an dem Vollhauttransplantat (44) befindlichen Schicht aus subkutanem Fettgewebe (46) ausgebildet und angeordnet ist, wobei die Vorrichtung (10) eine Zugwalze (16) umfasst, die über einen Spalt (34) beabstandet zu einer Schneide (32) der Schneideinrichtung (14) angeordnet ist und ausgebildet ist, um das Vollhauttransplantat (44) durch Rotation der Zugwalze (16) der Schneideinrichtung (14) zuzuführen, wobei die Schneideinrichtung (14) eine Klinge (30), die die Schneide (32) aufweist, umfasst, die an einem Klingenhalter (64) befestigt ist, **dadurch gekennzeichnet, dass** die Schneide (32) der Klinge (30) zur Rotationsachse (28) der Zugwalze (16) mit einem Schneidenabstand beabstandet ist und ein naheliegenster Abschnitt der Klinge (30) mit einem minimalen Abstand zur Rotationsachse (28) der Zugwalze (16) beabstandet ist, wobei der Schneidenabstand größer ist als der minimale Abstand.

2. Vorrichtung (10) nach Anspruch 1, **dadurch gekennzeichnet, dass** die Zugwalze (16) über eine Handkurbel (26) rotierbar ist.

3. Vorrichtung (10) nach Anspruch 1 oder 2, **dadurch gekennzeichnet, dass** die Zugwalze (16) über eine Antriebseinheit angetrieben rotierbar ist, wobei eine Rotationsgeschwindigkeit der Zugwalze (16) über ein Bedienelement im Betrieb der Vorrichtung (10) variierbar ist.

4. Vorrichtung (10) nach einem oder mehreren der vorangegangenen Ansprüche, **dadurch gekennzeichnet, dass** eine Schneide (32) der Schneideinrichtung (14)im Wesentlichen parallel zu einer Rotationsachse (28) der Zugwalze (16) angeordnet ist.

5. Vorrichtung (10) nach einem oder mehreren der vorangegangenen Ansprüche, **dadurch gekennzeichnet, dass** der Spalt (34) zwischen der Schneide (32) der Schneideinrichtung (14) und der Zugwalze (16) eine Spaltweite (38) von 0,8-1,2 mm aufweist.

6. Vorrichtung (10) nach einem oder mehreren der vorangegangenen Ansprüche, **dadurch gekennzeichnet, dass** die Zugwalze (16) auf ihrer Wirkfläche (42) rippenförmige und/oder knollige Erhebungen (40) aufweist.

7. Vorrichtung (10) nach einem oder mehreren der vorangegangenen Ansprüche, **dadurch gekennzeichnet, dass** die Spaltweite (38) zwischen der Schneide (32) und der Zugwalze (16) einstellbar ist.

8. Vorrichtung (10) nach einem oder mehreren der vorangegangenen Ansprüche, **dadurch gekennzeichnet, dass** die Zugwalze (16) jedenfalls bereichsweise eine verrundete Wirkfläche (42) aufweist, vorzugsweise auf ihrer gesamten Wirkfläche (42) verrundet ausgebildet ist.

9. Vorrichtung (10) nach einem oder mehreren der vorangegangenen Ansprüche, **dadurch gekennzeichnet, dass** die Zugwalze (16) auf ihrer Wirkfläche (42) wenigstens eine Ansaugöffnung (54), vorzugsweise eine Vielzahl an Ansaugöffnungen (54), die über die Wirkfläche (42) verteilt sind, aufweist, die mit einer Unterdruckquelle verbindbar ist bzw. sind.

10. Vorrichtung (10) nach einem oder mehreren der vorangegangenen Ansprüche, **dadurch gekennzeichnet, dass** die Vorrichtung einen Zuführtisch (63) zur Auflage des Vollhauttransplantats (44) mit daran befindlicher subkutaner Fettschicht (46) aufweist, über welchen das Vollhauttransplantat (44) der Zugwalze (16) zuführbar ist, vorzugsweise wobei beim Blick entlang der Rotationsachse (28) die Zugwalze (16) zwischen dem Zuführtisch (63) und der Schneide (32) angeordnet ist.

11. Verwendung einer Vorrichtung (10) nach einem oder mehreren der vorangegangenen Ansprüche zur Entfettung eines Vollhauttransplantats (44).

12. Verfahren zur Entfettung eines Vollhauttransplantats, wobei das Verfahren unter Verwendung einer Vorrichtung (10) gemäß einem oder mehreren der vorangegangenen Ansprüche 1 bis 10 durchgeführt wird, wobei das Verfahren die Schritte umfasst:
das Vollhauttransplantat (44) mit der daran befindlichen subkutanen Fettschicht (46) derart auf eine Zugwalze (16) aufbringen, dass das Vollhauttransplantat (44) der Zugwalze (16) zugewandt ist und die subkutane Fettschicht (46) der Zugwalze (16) abgewandt ist;
das Vollhauttransplantat (44) mit der daran befindlichen subkutanen Fettschicht (46) durch Rotation der Zugwalze (16) gegen eine Schneide (32) einer Schneideeinrichtung (14) führen, wobei ein Spalt (34) zwischen der Zugwalze (16) und der Schneideinrichtung (14) derart ausgebildet ist, dass das Vollhauttransplantat (44) durch die Rotation der Zugwalze (16) zwischen der Zugwalze (16) und der Schneideeinrichtung (14) in den Spalt (34) geführt wird, während die Schneideinrichtung (14) die subkutane Fettschicht (46) von dem Vollhauttransplantat (44) abtrennt.

13. Verfahren nach dem voranstehenden Anspruch, **dadurch gekennzeichnet, dass** die Rotation der Zugwalze (16) manuell, insbesondere über eine Handkurbel (26) erfolgt.

## Claims

1. Device (10) for defatting a full-thickness skin graft (44), having a cutting means (14) which is configured and arranged for separating a layer of subcutaneous fatty tissue (46) located on the full-thickness skin graft (44), wherein the device (10) has a pull roller (16) which is arranged at a distance from a cutting edge (32) of the cutting means (14) across a gap (34) and is configured to feed the full-thickness skin graft (44) to the cutting means (14) by rotation of the pull roller (16), wherein the cutting means (14) comprises a blade (30) having the cutting edge (32), which blade is attached to a blade holder (64), **characterized in that** the cutting edge (32) of the blade (30) is spaced apart from the axis of rotation (28) of the pull roller (16) with a cutting edge spacing and a closest portion of the blade (30) is spaced a minimum distance from the axis of rotation (28) of the pull roller (16), the cutting edge spacing being greater than the minimum distance.

2. Device (10) according to claim 1, **characterized in that** the pull roller (16) is rotatable via a hand crank (26).

3. Device (10) according to claim 1 or claim 2, **characterized in that** the pull roller (16) can be driven in rotation by a drive unit, the rotational speed of the pull roller (16) being variable via a control element during operation of the device (10).

4. Device (10) according to one or more of the preceding claims, **characterized in that** a cutting edge (32) of the cutting means (14) is arranged substantially parallel to an axis of rotation (28) of the pull roller (16).

5. Device (10) according to one or more of the preceding claims, **characterized in that** the gap (34) between the cutting edge (32) of the cutting means (14) and the pull roller (16) has a gap width (38) of 0.8-1.2 mm.

6. Device (10) according to one or more of the preceding claims, **characterized in that** the pull roller (16) has rib-shaped and/or nodular elevations (40) on its active surface (42).

7. Device (10) according to one or more of the preceding claims, **characterized in that** the gap width (38) between the cutting edge (32) and the pull roller (16) is adjustable.

8. Device (10) according to one or more of the preceding claims, **characterized in that** the pull roller (16) has a rounded active surface (42) at least in certain regions, preferably is rounded over its entire active surface (42) .

9. Device (10) according to one or more of the preceding claims, **characterized in that** the pull roller (16) has on its active surface (42) at least one suction opening (54), preferably a large number of suction openings (54) which are distributed over the active surface (42), which is or are connectable to a vacuum source.

10. Device (10) according to one or more of the preceding claims, **characterized in that** the device has a feed table (63) for supporting the full-thickness skin graft (44) with the subcutaneous fat layer (46) located thereon, via which the full-thickness skin graft (44) can be fed to the pull roller (16), preferably with the pull roller (16) being arranged between the feed table (63) and the cutting edge (32) when viewed along the axis of rotation (28).

11. Use of a device (10) according to one or more of the preceding claims for defatting a full-thickness skin graft (44).

12. Method for defatting a full-thickness skin graft, the method being carried out using a device (10) according to one or more of the preceding claims 1 to 10, the method comprising the following steps:
applying the full-thickness skin graft (44) with the subcutaneous fat layer (46) thereon to a pull roller (16) in such a way that the full-thickness skin graft (44) faces the pull roller (16) and the subcutaneous fat layer (46) faces away from the pull roller (16);
guiding the full-thickness skin graft (44) with the subcutaneous fat layer (46) thereon by rotating the pull roller (16) against a cutting edge (32) of a cutting means (14), a gap (34) between the pull roller (16) and the cutting means (14) being configured in such a way that the full-thickness skin graft (44) is guided into the gap (34) between the pull roller (16) and the cutting means (14) by the rotation of the pull roller (16), while the cutting means (14) separates the subcutaneous fat layer (46) from the full-thickness skin graft (44).

13. Method according to the preceding claim, **characterized in that** the pull roller (16) is rotated manually, in particular by means of a hand crank (26).

## Revendications

1. Dispositif (10) pour dégraisser un greffon de peau entière (44), avec un système de coupe (14), qui est réalisé et disposé pour séparer une couche de tissu graisseux sous-cutané (46) se trouvant sur le greffon de peau entière (44), dans lequel le dispositif (10) comprend un rouleau de traction (16), qui est disposé au-dessus d'une fente (34) à distance d'un tranchant (32) du système de coupe (14) et est réalisé pour amener le greffon de peau entière (44) au système de coupe (14) par rotation du rouleau de traction (16), dans lequel le système de coupe (14) présente une lame (30), qui présente le tranchant (32), qui est fixé à un porte-lame (64), **caractérisé en ce que** le tranchant (32) de la lame (30) est espacé de l'axe de rotation (28) du rouleau de traction (16) avec une distance de tranchant et une partie la plus proche de la lame (30) est espacée avec une distance minimale de l'axe de rotation (28) du rouleau de traction (16), dans lequel la distance de tranchant est supérieure à la distance minimale.

2. Dispositif (10) selon la revendication 1, **caractérisé en ce que** le rouleau de traction (16) peut être amené en rotation par l'intermédiaire d'une manivelle (26).

3. Dispositif (10) selon la revendication 1 ou 2, **caractérisé en ce que** le rouleau de traction (16) peut être amené en rotation en étant entraîné par l'intermédiaire d'une unité d'entraînement, dans lequel une vitesse de rotation du rouleau de traction (16) peut être modifiée par l'intermédiaire d'un élément de commande utilisateur lors du fonctionnement du dispositif (10).

4. Dispositif (10) selon l'une ou plusieurs des revendications précédentes, **caractérisé en ce qu'**un tranchant (32) du système de coupe (14) est disposé sensiblement parallèlement à un axe de rotation (28) du rouleau de traction (16) .

5. Dispositif (10) selon l'une ou plusieurs des revendications précédentes, **caractérisé en ce que** la fente (34) entre le tranchant (32) du système de coupe (14) et le rouleau de traction (16) présente une largeur de fente (38) de 0,8-1,2 mm.

6. Dispositif (10) selon l'une ou plusieurs des revendications précédentes, **caractérisé en ce que** le rouleau de traction (16) présente sur sa surface active (42) des élévations (40) en forme de nervure et/ou bulbeuses.

7. Dispositif (10) selon l'une ou plusieurs des revendications précédentes, **caractérisé en ce que** la largeur de fente (38) entre le tranchant (32) et le rouleau de traction (16) est réglable.

8. Dispositif (10) selon l'une ou plusieurs des revendications précédentes, **caractérisé en ce que** le rouleau de traction (16) présente dans tous les cas par endroits une surface active (42) arrondie, de préférence est réalisé de manière arrondie sur toute sa surface active (42).

9. Dispositif (10) selon l'une ou plusieurs des revendications précédentes, **caractérisé en ce que** le rouleau de traction (16) présente sur sa surface active (42) au moins une ouverture d'aspiration (54), de préférence une pluralité d'ouvertures d'aspiration (54), qui sont réparties sur la surface active (42), qui peut ou peuvent être reliée (s) à une source de vide.

10. Dispositif (10) selon l'une ou plusieurs des revendications précédentes, **caractérisé en ce que** le dispositif présente une table d'amenée (63) pour le support du greffon de peau entière (44) avec une couche graisseuse sous-cutanée (46) se trouvant sur celui-ci, par l'intermédiaire de laquelle le greffon de peau entière (44) peut être amené au rouleau de traction (16), de préférence dans lequel en regardant le long de l'axe de rotation (28) le rouleau de traction (16) est disposé entre la table d'amenée (63) et le tranchant (32).

11. Utilisation d'un dispositif (10) selon l'une ou plusieurs des revendications précédentes pour le dégraissage d'un greffon de peau entière (44).

12. Procédé pour le dégraissage d'un greffon de peau entière, dans lequel le procédé est mis en œuvre au moyen d'un dispositif (10) selon l'une ou plusieurs des revendications 1 à 10 précédentes, dans lequel le procédé comprend les étapes consistant à :
appliquer le greffon de peau entière (44) avec la couche graisseuse sous-cutanée (46) se trouvant sur celui-ci sur un rouleau de traction (16), de telle sorte que le greffon de peau entière (44) est tourné vers le rouleau de traction (16) et la couche graisseuse sous-cutanée (46) est opposée au rouleau de traction (16) ;
guider le greffon de peau entière (44) avec la couche graisseuse sous-cutanée (46) se trouvant sur celui-ci par rotation du rouleau de traction (16) contre un tranchant (32) d'un système de coupe (14), dans lequel une fente (34) entre le rouleau de traction (16) et le système de coupe (14) est réalisée de telle sorte que le greffon de peau entière (44) est guidé par la rotation du rouleau de traction (16) entre le rouleau de traction (16) et le système de coupe (14) dans la fente (34), pendant que le système de coupe (14) sépare la couche graisseuse sous-cutanée (46) du greffon de peau entière (44) .

13. Procédé selon la revendication précédente, **caractérisé en ce que** la rotation du rouleau de traction (16) s'effectue manuellement, en particulier par l'intermédiaire d'une manivelle (26).
